(19) Europäisches Patentamt
European Patent Office
Office européen des brevets

(11) Veröffentlichungsnummer: **0 049 776**
**B1**

(12) # EUROPÄISCHE PATENTSCHRIFT

(45) Veröffentlichungstag der Patentschrift: **31.07.85**

(21) Anmeldenummer: **81107340.2**

(22) Anmeldetag: **17.09.81**

(51) Int. Cl.[4]: **C 07 D 401/06,** C 07 D 215/12, C 07 D 215/18, C 07 F 9/60 // C07D213/57, C07F9/58, C07D401/14, A61K31/47

(54) **Neue 4-Chinolinmethanderivate, ihre Herstellung und Verwendung zur Darstellung von Substanzen mit Arzneimittelwirkung.**

(30) Priorität: **11.10.80 DE 3038504**

(43) Veröffentlichungstag der Anmeldung: **21.04.82 Patentblatt 82/16**

(45) Bekanntmachung des Hinweises auf die Patenterteilung: **31.07.85 Patentblatt 85/31**

(84) Benannte Vertragsstaaten: **BE CH DE FR GB IT LI LU NL**

(56) Entgegenhaltungen: **JOURNAL OF LABELLED COMPOUNDS AND RADIOPHARMACEUTICALS, Band 17, Nr. 3, 1980, Seiten 431-437 New York, U.S.A. W.H. YANKO et al.: "C-labeled antimalarials. I. Synthesis of d1-crythro- and threo-alpha-(2-piperidyl)-2,8-bis(trifluoromethyl)-4-quinolinemethanol-alpha-14C"**

(73) Patentinhaber: **BASF Aktiengesellschaft, Carl-Bosch-Strasse 38, D-6700 Ludwigshafen (DE)**

(72) Erfinder: **Hickmann, Eckhard, Dr., Kantstrasse 23, D-6701 Dannstadt-Schauernheim (DE)**
Erfinder: **Oeser, Heinz-Guenter, Dr., Mohngasse 25, D-6711 Dirmstein (DE)**

EP 0 049 776 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 4-Chinolinmethanderivate, deren Herstellung sowie deren Verwendung zur Darstellung von 4-Chinolinmethanolderivaten, welche eine Arzneimittelwirkung, insbesondere gegen Malaria, besitzen.

Bestimmte 4-Chinolinmethanolderivate, z. B. das Mefloquin (d,1-erythro-α-(2-Piperidyl)-2,8-bis-(trifluormethyl)-4-chinolinmethanol (vgl. zum Beispiel Antimicrobial Agents Chemother. 9, 384 (1976)), sind wertvolle Wirkstoffe zur Bekämpfung von Malaria. Diese Substanzen werden bislang über metallorganische Zwischenstufen hergestellt (vgl. J. Med. Chem. 14, 926 (1971); DOS 2 806 909; Journal of labelled Compounds and Radiopharmaceuticals 17, 431 (1980)), was für die Herstellung in größerem Maßstab sehr nachteilig ist.

Es wurde nun gefunden, daß die neuen 4-Chinolinmethan-Derivate der Formel I

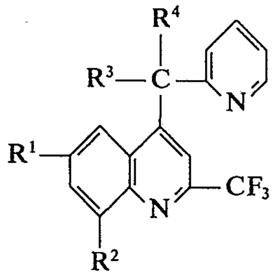

(I)

worin

$R^1$ ein Wasserstoffatom oder ein Chloratom,
$R^2$ eine Trifluormethylgruppe oder ein Chloratom,
$R^3$ eine Cyano- oder Triphenylphosphoranmethino-Gruppe und
$R^4$ ein Wasserstoffatom oder einen Acetalrest mit bis zu 8 Kohlenstoffatomen

darstellen, sehr einfach hergestellt werden können und wertvolle Zwischenprodukte für die Herstellung von 4-Chinolinmethanol-Derivaten darstellen.

Die erfindungsgemäßen 4-Chinolinmethanderivate der Formel I werden hergestellt, indem man Verbindungen der Formel II,

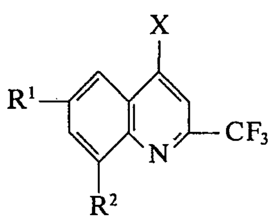

(II)

in der $R^1$ und $R^2$ die oben angegebene Bedeutung haben und in der X eine elektrofuge Gruppe bedeutet, mit einer Verbindung der Formel III,

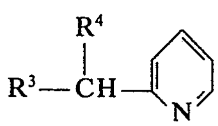

(III)

in der $R^3$ und $R^4$ die oben angegebenen Bedeutungen haben, in einem inerten Lösungsmittel in Gegenwart einer Base umsetzt.

Als elektrofuge Gruppe X in Formel II eignet sich besonders Chlor, Brom oder Jod.

Als Lösungsmittel für die Umsetzung der Verbindungen II mit den Verbindungen III kommen unter den Reaktionsbedingungen inerte organische Lösungsmittel in Betracht, wie aromatische Kohlenwasserstoffe, wie Benzol, Toluol oder Xylole, gesättigte aliphatische und cyclische Ether, wie Diethylether, Diisopropylether, Dimethoxyethan, Diethylenglykoldimethylether, Methyl-tert.-butylether, Tetrahydrofuran, Dioxan, Alkohole, wie Methanol und tert.-Butanol, tertiäre Amine und Trialkylamine, wie Pyridin oder Triethylamin, sowie aprotische, stark polare Lösungsmittel, insbesondere Acetonitril, Dimethylformamid und Dimethylsulfoxid.

In vielen Fällen läßt sich die Reaktion vorteilhafterweise in einem zweiphasigen System mit einem Phasentransferkatalysator durchführen. Die organische Phase wird dabei von einem der obengenannten Lösungsmittel gebildet, während die wäßrige Phase zweckmäßigerweise eine wäßrige Lösung von Natrium- oder Kaliumhydroxid oder auch Natrium- oder Kaliumcarbonat ist. Als Phasentransferkatalysatoren kommen die üblicherweise verwendeten quaternären Ammonium- oder Phosphoniumsalze in Betracht, wie Triethyl-benzyl-ammoniumchlorid, Hexadecyl-trimethyl-ammoniumchlorid, Tricaprylmethyl-ammoniumchlorid bzw. -bromid oder Hexadecyl-tributyl-phosphoniumbromid.

Die Umsetzung von II mit III erfolgt unter Basenkatalyse. Als Basen kommen beispielsweise in Betracht: Alkali- und Erdalkalihydroxide und -carbonate, wie Natrium-, Kalium- oder Calciumhydroxid, Natrium- oder Kaliumcarbonat, Alkalialkoholate, insbesondere Methylate, Ethylate, Isopropylate oder tert.-Butylate des Natriums und Kaliums und Alkaliamide, wie Natrium- oder Kaliumamid oder Natriumhydrid.

Zweckmäßigerweise paßt man die Basizität der verwendeten Base der Acidität der CH-aciden, nucleophilen Komponente III an. So kann man beispielsweise bei Verwendung von 2-Pyridyl-acetonitril vorteilhaft wäßrige Natriumhydroxidlösung oder eine alkoholische Alkoholatlösung einsetzen.

Das Mengenverhältnis der CH-aciden Verbindung III zur Verbindung II liegt normalerweise nahe bei oder genau bei einem Molverhältnis von 1 : 1.

Das Mengenverhältnis der Base zur CH-aciden Verbindung III beträgt vorzugsweise 2 Mol pro Mol.

Die Reaktionen werden üblicherweise in dem Temperaturbereich von ca. $-20°C$ bis zum Siedepunkt des jeweiligen Lösungsmittels durchgeführt. Bevorzugt ist der Temperaturbereich von ca. 20°C bis ca. 60°C. Es ist zweckmäßig, unter Inertgasatmosphäre zu arbeiten. Als Schutzgase eignen sich besonders Stickstoff und Argon.

Die neuen Verbindungen eignen sich sehr gut zur Darstellung von 4-Chinolinmethanol-Derivaten, die eine gute Wirkung gegen Malaria besitzen. Diese Derivate können aus den neuen Verbindungen in ausgezeichneter Ausbeute und in hoher Reinheit gewonnen werden. Darüber hinaus eröffnen die neuen Verbindungen wesentlich einfachere und sichere Wege zur Herstellung der 4-Chinolinmethanol-Derivate als die, die z. B. in J. Med. Chem. 14, 926 (1971), DE-OS 2 806 909, DE-OS 2 940 443 beschrieben sind und für die metallorganische Reagentien oder Grignard-Verbindungen benötigt werden.

Die neuen Verbindungen brauchen für die weiteren Umsetzungen in den meisten Fällen nicht besonders gereinigt zu werden. Häufig kann das die Verbindung enthaltende Reaktionsgemisch direkt weiter verarbeitet werden.

Wie die Ausführungsbeispiele zeigen, werden aus den erfindungsgemäßen 4-Chinolin-methanderivaten die gewünschten 4-Chinolinmethanolderivate in ausgezeichneten Ausbeuten und in hoher Reinheit gewonnen.

Das 2-Pyridyl-2,8-bis-(trifluormethyl)-4-chinolincyanomethan kann nicht in Analogie zu einer Literaturvorschrift (Y. Masuyama et. al., Chem. Letters, 1439 (1977)) in einem Zweiphasensystem oxydativ decyaniert werden, da anstelle des erwarteten Pyridylketons das 2,8-Bis-(trifluormethyl)-chinolin entsteht. Überraschenderweise wurde jedoch gefunden, daß der Abbau zum Pyridylketon mit fast quantitativen Ausbeuten gelingt, wenn man die Reaktion in polaren wasserfreien Medien, z. B. aliphatischen Alkoholen, bevorzugt Methanol oder tert.-Butanol, Dimethylformamid, Dimethylsulfoxyd, Hexamethylphosphorsäuretriamid oder Pyridin, in Gegenwart von Basen, z. B. Alkoholaten, bevorzugt Natriummethylat oder Kalium-tert.-butylat, tertiären Aminen, z. B. Triethylamin oder Diaza-bicyclo-[2.2.2]-octan, anorganischen Basen, z. B. Calciumhydroxid oder Kaliumcarbonat, unter Begasen mit Sauerstoff oder sauerstoffhaltigen Gasen, bevorzugt Luft, bei Raumtemperatur oder erhöhter Temperatur durchführt. Eine besonders bevorzugte Ausführungsform besteht darin, das 2-Pyridyl-2,8-bis-(trifluormethyl)-4-chinolin-cyanomethan in tert.-Butanol als Lösungsmittel in Gegenwart von Kalium-tert.-butylat als Base unter einer Inertgasatmosphäre aus 2,8-Bis-(trifluormethyl)-4-chlorchinolin und 2-Pyridylnitril zu erzeugen und ohne Isolierung bei Temperaturen oberhalb von ca. 35°C durch intensives Begasen mit Luft in das Pyridylketon umzuwandeln. Überraschend gut kann das 2-Pyridyl-2,8-bis-(trifluormethyl)-4-chinolin-cyanomethan auch in saurem Medium, z. B. in Essigsäure, mit Oxydationsmitteln, z. B. Wasserstoffperoxid oder Peressigsäure, zum Pyridylketon umngewandelt werden.

Das 2-Pyridyl-2,8-bis-(trifluormethyl)-4-chinolin($\alpha$-ethoxy)-ethoxy-methan und analoge Cyanhydrinacetale liefern nach säurekatalysierter Abspaltung des Acetalrestes und Alkalibehandlung des erhaltenen Cyanhydrins das Pyridylketon.

Das 2-Pyridyl-2,8-bis-(trifluormethyl)-4-chinolyl-methino-triphenylphosphan, das als Ylid vorliegt, wird durch Behandeln mit sauerstoffhaltigen Oxydationsmitteln unter Abspaltung von Triphenylphosphinoxid in das Pyridylketon übergeführt.

Das 2-Pyridyl-2-trifluormethyl-6,8-dichlor-4-chinolin-cyanomethan kann in Analogie zum 2-Pyridyl-2,8-bis-(trifluormethyl)-4-chinolin-cyanomethan in das 2-Pyridyl-6,8-dichlor-4-chinolylketon überführt weden, dessen katalytische Hydrierung das gewünschte $\alpha$-(2-Piperidyl)-2-trifluormethyl-6,8-dichlorchinolinmethanol ergibt.

Die folgenden Beispiele erläutern die Erfindung näher. In ihnen verhalten sich Volumenteile zu Teilen wie Liter zu Kilogramm.

## I. Herstellung der neuen Verbindungen

### Beispiel 1

Man versetzt das Gemisch aus 62 Teilen 2,8-Bis-(trifluormethyl)-4-bromchinolin, 106 Volumenteilen Dimethylformamid (DMF), 1,7 Teilen Tetrabutyl-ammoniumchlorid und 20,9 Teilen 2-Pyridylacetonitril bei Raumtemperatur unter Stickstoffbegasung mit 35,5 Teilen konz. Natronlauge und rührt das Reaktionsgemisch noch eine Stunde. Anschließend wird das Reaktionsprodukt mit ca. 200 Volumenteilen Wasser versetzt und mit Eisessig auf pH 4 eingestellt. Das ausgefallene Produkt wird abgesaugt, mit Wasser gründlich gewaschen und getrocknet. Man erhält 67 Teile = 97,6% d. Th. 2-Pyridyl-2,8-bis-(trifluormethyl)-4-chinolin-cyanomethan vom Fp. 162 bis 165° C. Eine analysenreine Probe schmilzt bei 167 bis 168° C.

### Beispiel 2

a) Herstellung des Ausgangsmaterials

Das Gemisch aus 100 Volumenteilen Phosphortrichlorid und 40 Teilen 2,8-Bis-(trifluormethyl)-4-hydroxychinolin wird 8 Stunden unter Rückfluß erhitzt. Anschließend destilliert man den größten Teil des überschüssigen Phosphortrichlorids ab, gießt den Rückstand auf 200 Teile Eiswasser und stellt mit 12 n Natronlauge auf pH 12 bis 13. Anschließend wird mit Dichlormethan extrahiert, über Natriumsulfat getrocknet und das Lösungsmittel unter reduziertem Druck abgezogen. Man erhält 40,2 Teile = 94% d. Th. 2,8-Bis-(trifluormethyl)-4-chlorchinolin vom Fp. 40—42° C.

b) Herstellung des 4-Chinolinmethanderivats

Analog Beispiel 1 erhält man unter Verwendung von 54 Teilen des gemäß 2a) erhaltenen Produkts 63,5 Teile = 88% d. Th. 2-Pyridyl-2,8-bis-(trifluormethyl)-4-chinolin-cyanomethan vom Fp. 162—164° C.

### Beispiel 3

Das Gemisch aus 250 Volumenteilen tert.-Butanol, 44,9 Teilen Kalium-tert.-butylat und 57,1 Teilen 2,8-Bis-(trifluormethyl)-4-chlorchinolin wird bei Raumtemperatur unter Rühren mit 23,6 Teilen 2-Pyridyl-acetonitril versetzt und unter Stickstoff ca. 1,5 Stunden gerührt. Das Reaktionsprodukt wird mit 12,0 Teilen Essigsäure versetzt und mit ca. 300 Volumenteilen Wasser verdünnt. Das ausgefallene Produkt wird mit Wasser gründlich gewaschen und bei ca. 50° C im Vakuumtrockenschrank getrocknet. Man erhält 74,3 Teile = 97,5% d. Th. 2-Pyridyl-2,8-bis-(trifluormethyl)-4-chinolin-cyanomethan (vgl. Beispiel 1) vom Fp. 163 bis 165° C.

### Beispiel 4

Das Gemisch aus 200 Volumenteilen DMF, 17,2 Teilen 2,8-Bis-(trifluormethyl)-4-bromchinolin, 30,4 Teilen fein pulverisiertem Kaliumcarbonat und 9,0 Teilen 2-Picolylchlorid-hydrochlorid wird 2 Stunden bei Raumtemperatur unter Stickstoff gerührt. Anschließend gibt man 5,4 Teile Kaliumcyanid portionsweise zu, erhöht die Temperatur auf 50° C und rührt weitere 16 Stunden. Das Reaktionsgemisch wird vom Ungelösten abfiltriert, mit 3 Teilen Eisessig neutralisiert und mit 300 ml Wasser verdünnt. Das ausgefallene Produkt wird abgesaugt, mit Wasser gewaschen und im Vakuumtrockenschrank bei ca. 50° C getrocknet. Man erhält 18 Teile = 95% rohes 2-Pyridyl-2,8-bis-(trifluormethyl)-4-chinolin-cyanomethan (vgl. Beispiel 1), das als Nebenprodukt etwas 2-Pyridyl-2-picolyl-2,8-bis-(trifluormethyl)-4-chinolin-cyanomethan enthält.

### Beispiel 5

Das Gemisch aus 250 Volumenteilen tert.-Butanol, 20,6 Teilen 2,8-Bis-(trifluormethyl)-4-bromchinolin und 7,1 Teilen 2-Pyridyl-acetonitril wird mit 13,5 Teilen Kalium-tert.-butylat unter Stickstoff versetzt und ca. 1,5 Stunden bei Raumtemperatur gerührt. Das so erhaltene Reaktionsgemisch enthält 2-Pyridyl-2,8-bis-(trifluormethyl)-4-chinolin-cyanomethan (vgl. Beispiel 1) in Form seines intensiv permanganatfarbenen Kaliumsalzes. Das Gemisch kann direkt weiterverwendet werden (s. Beispiel G).

Beispiel 6

a) Herstellung des Ausgangsmaterials

Das Gemisch aus 190 Volumenteilen Methylenchlorid, 17 Teilen rohem 2-Pyridinaldehydcyanhydrin, 10 Teilen Vinylethylether und 0,3 Teilen p-Toluolsulfonsäure wird 1,5 Stunden bei 40°C gerührt. Anschließend gibt man 5 Teile pulverisiertes Kaliumcarbonat zu, rührt eine weitere Stunde, filtriert die organische Phase ab, wäscht sie mit Wasser und zieht das organische Lösungsmittel ab. Der Rückstand wird zwischen 100 Volumenteilen Toluol und 10 Volumenteilen 2 N Natronlauge verteilt, die organische Phase wird eingedampft (5,3 Teile Rückstand) und unter reduziertem Druck destilliert. Man erhält 3,3 Teile 2-Pyridyl-($\alpha$-ethoxy)-ethoxy-acetonitril vom $Kp_{0,3} = 80-85°C$.

b) Herstellung des 4-Chinolinmethanderivates

Das Gemisch aus 10,6 Teilen 2-Pyridyl-($\alpha$-ethoxy)-ethoxy-acetonitril, 17,3 Teilen 2,8-Bis-(trifluormethyl)-4-bromchinolin, 0,5 Teilen Tetrabutyl-ammoniumchlorid und 50 Volumenteilen Dimethylsulfoxid wird bei Raumtemperatur unter Stickstoff und unter Rühren langsam mit dem Gemisch aus 10 Teilen konz. Natronlauge und 20 Teilen Wasser versetzt; dabei steigt die Temperatur auf ca. 28°C an. Nach einer halben Stunde Reaktionsdauer sind die Ausgangsstoffe dünnschichtchromatographisch nicht mehr nachweisbar. Man verdünnt den Ansatz mit 200 Teilen Wasser und extrahiert das Produkt dreimal mit je 50 Volumenteilen Toluol. Die vereinigten Toluolextrakte werden einmal mit 100 Teilen Wasser gewaschen und eingedampft. Man erhält 19,5 Teile = 82,5% d. Th. 2-Pyridyl-2,8-bis-(trifluormethyl)-4-chinolin-($\alpha$-ethoxy)-ethoxy-methan als Öl mit zutreffendem Massenspektrum (Molpeak bei 469).

Beispiel 7

a) Herstellung des Ausgangsmaterials

Das Gemisch aus 60 Volumenteilen DMF, 6,4 Teilen 2-Picolylchlorid und 13,2 Teilen Triphenylphosphin wird unter Stickstoff 4 Stunden bei 100°C gerührt. Die ausgefallenen Kristalle werden abgesaugt, mit Petrolether gewaschen und getrocknet. Man erhält 12 Teile = 59% d. Th. Triphenyl-2-picolylphosphoniumchlorid.

b) Herstellung des 4-Chinolinmethanderivats

Das Gemisch aus 50 Volumenteilen DMF, 34,4 Teilen 2,8-Bis-(trifluormethyl)-4-bromchinolin, 40,4 Teilen Triphenyl-2-picolyl-phosphoniumchlorid und 110 Teilen Kaliumcarbonat wird unter Stickstoff 3 Stunden gerührt, wobei die Reaktionstemperatur zuletzt auf 155°C gesteigert wird. Das Reaktionsgemisch wird mit ca. 200 Volumenteilen Wasser verdünnt und zweimal mit je 100 Volumenteilen Toluol extrahiert. Der Toluolextrakt wird eingedampft und aus Toluol/Hexan umgefällt. Man erhält 56 Teile = 89% d. Th. 2-Pyridyl-2,8-bis-(trifluormethyl)-4-chinolyl-methino-triphenylphosphan vom Fp. 153 bis 155°C. Das Produkt besitzt im NMR-Spektrum ein Singulett bei $\delta = 7,28$ ppm (Proton in 3-Stellung am Chinolinring). Das Massenspektrum zeigt den Molpeak bei 627.

Beispiel 8

Das Gemisch aus 800 Volumenteilen DMF, 85,6 Teilen 2-Pyridyl-acetonitril und 250 Teilen 2-Trifluormethyl-6,8-dichlor-4-bromchinolin wird mit einem Teil Tetrabutylammoniumchlorid und, unter Kühlung bei Raumtemperatur, mit 150 Teilen konz. Natronlauge versetzt und 1,5 Stunden unter Stickstoff gerührt. Man trenn die DMF-Phase ab, versetzt sie mit 45 Teilen Eisessig und verdünnt mit 1000 Volumenteilen Wasser. Das ausgefallene Produkt wird abgesaugt, mit Wasser gewaschen und im Vakuumtrockenschrank bei ca. 50°C getrocknet. Man erhält 255 Teile = 92% d. Th. 2-Pyridyl-2-trifluormethyl-6,8-dichlor-4-chinolin-cyanomethan vom Fp. 171—173°C.

Beispiel 9

a) Herstellung des Ausgangsmaterials

Man verfährt analog Beispiel 2a), setzt jedoch anstelle von 2,8-Bis-(trifluormethyl)-4-hydroxychinolin 40 Teile 2-Trifluormethyl-6,8-dichlor-4-hydroxychinolin ein. So erhält man 38,9 Teile = 91% d. Th. 2-Trifluormethyl-4,6,8-trichlorchinolin vom Fp. 74—76°C.

b) Herstellung des 4-Chinolinmethanderivats

Man verfährt wie in Beispiel 3 beschrieben, setzt jedoch anstelle von 2,8-Bis-(trifluormethyl)-4-chlorchinolin 57,3 Teile 2-Trifluormethyl-4,6,8-trichlorchinolin ein. So erhält man 69,5 Teile = 95% d. Th. 2-Pyridyl-2-trifluormethyl-6,8-dichlor-4-chinolin-cyanomethan vom Fp. 172—173° C.

II. Verwendung der neuen Verbindungen

Beispiel A

In die Lösung von 40 Teilen 2-Pyridyl-2,8-bis-(trifluormethyl)-4-chinolin-cyanomethan (Beispiel 3) und 12,6 Teilen Kalium-tert.-butylat in 200 Volumenteilen tert.-Butanol wird bei Raumtemperatur 3 Stunden Sauerstoff eingeleitet. Anschließend wird das Reaktionsprodukt mit ca. 1000 Volumenteilen Wasser versetzt und dreimal mit je 500 Volumenteilen Toluol extrahiert. Der Toluolextrakt wird mit Wasser gewaschen und eingedampft. Man erhält 38 Teile = 97,8% d. Th. 2-Pyridyl-2,8-bis-(trifluormethyl)-4-chinolylketon vom Fp. 114—118° C.

Beispiel B

In das Gemisch aus 4,0 Teilen 2-Pyridyl-2,8-bis-(trifluormethyl)-4-chinolin-cyanomethan (Beispiel 1), 4,7 Teilen 30%iger methanolischer Natriummethylatlösung und 10 Volumenteilen Methanol wird 9 Stunden bei 40° C und weitere 5 Stunden bei 50° C unter Rühren Sauerstoff eingeleitet. Das Reaktionsprodukt wird mit 100 Volumenteilen Wasser versetzt und dreimal mit je 100 Volumenteilen Toluol extrahiert. Die vereinigten organischen Phasen werden zur Trockene eingeengt. Man erhält 3,4 Teile = 88% d. Th. dünnschichtchromatographisch reines 2-Pyridyl-2,8-bis-(trifluormethyl)-4-chinolylketon.

Beispiel C

In das Gemisch aus 4,0 Teilen 2-Pyridyl-2,8-bis-(trifluormethyl)-4-chinolin-cyanomethan (Beispiel 1), 1,46 Teilen Diaza-bicyclo-[2,2,2]-octan und 20 Volumenteilen Dimethylformamid wird 10 Stunden bei 50° C und weitere 2 Stunden bei 60° C unter Rühren Sauerstoff eingeleitet. Nach der in Beispiel B beschriebenen Aufarbeitung erhält man 2,9 Teile = 64% d. Th. 2-Pyridyl-2,8-bis-(trifluormethyl)-4-chinolylketon.

Beispiel D

In das Gemisch aus 4,0 Teilen 2-Pyridyl-2,8-bis-(trifluormethyl)-4-chinolin-cyanomethan (Beispiel 1), 1,0 Teilen Calciumhydroxid und 20 Volumenteilen Dimethylformamid wird 17 Stunden bei 50° C unter Rühren Sauerstoff eingeleitet. Nach der unter Beispiel B beschriebenen Aufarbeitung erhält man 3,2 Teile = 82% d. Th. 2-Pyridyl-2,8-bis-(trifluormethyl)-4-chinolylketon.

Beispiel E

Das Gemisch aus 250 Volumenteilen tert.-Butanol, 7,07 Teilen Kalium-tert.-butylat und 22,9 Teilen 2-Pyridyl-2,8-bis-(trifluormethyl)-4-chinolin-cyanomethan (Beispiel 1) wird auf 60° C erwärmt. Bei dieser Temperatur wird unter intensiver Durchmischung Luft eingeleitet, bis das Nitril umgesetzt ist. Man setzt 3,8 Teile Eisessig zu und verdünnt das Reaktionsprodukt mit 300 Volumenteilen Wasser. Das ausgefallene Produkt wird abfiltriert, mit Wasser gewaschen und bei 50° C im Vakuumtrockenschrank getrocknet. Man erhält 21,6 Teile = 97% d. Th. 2-Pyridyl-2,8-bis-(trifluormethyl)-4-chinolylketon vom Fp. 128—129° C.

Beispiel F

Die Lösung von 5,0 Teilen 2-Pyridyl-2,8-bis-(trifluormethyl)-4-chinolin-cyanomethan in 20 Volumenteilen Eisessig wird auf 110° C erhitzt und innerhalb von 5 Minuten mit 1,1 Teilen 50%igem Wasserstoffperoxid versetzt. Anschließend rührt man noch eine Stunde bei Siedetemperatur. Der Überschuß an Wasserstoffperoxid wird durch Zugabe von Natriumsulfit zerstört und das Reaktionsgemisch nach Zugabe von 50 Volumenteilen Wasser zweimal mit je 100 Volumenteilen Toluol extrahiert. Durch Eindampfen der Toluollösung erhält man 4,1 Teile = 84% d. Th. 2-Pyridyl-2,8-bis-(trifluormethyl)-4-chinolylketon.

Beispiel G

Man erwärmt das gemäß Beispiel 5 erhaltene Gemisch auf 50 bis 60°C und leitet ca. 15 Stunden unter intensivem Durchmischen Luft durch. Anschließend gibt man 3,6 Teile Eisessig zu und verdünnt das Reaktionsgemisch mit 300 Volumenteilen Wasser. Das ausgefallene Produkt wird mit Wasser gründlich gewaschen und bei ca. 50°C im Vakuumtrockenschrank getrocknet. Man erhält 21,3 Teile = 96% d. Th. 2-Pyridyl-2,8-bis-(trifluormethyl)-4-chinolylketon vom Fp. 127—128°C.

Beispiel H

Das Gemisch aus 100 Volumenteilen Methanol, 4,7 Teilen 2-Pyridyl-2,8-bis-(trifluormethyl)-4-chinolin-($\alpha$-ethoxy)-ethoxymethan (Beispiel 6) und 1 Teil konz. Salzsäure wird 3 Stunden bei Raumtemperatur gerührt. Anschließend gibt man 5 Volumenteile 6 N Natronlauge und 200 Volumenteile Wasser zu, rührt noch 0,5 Stunden, saugt das ausgefallene Produkt ab, wäscht es mit Wasser und trocknet es. Man erhält 2,5 Teile = 68% d. Th. 2-Pyridyl-2,8-bis-(trifluormethyl)-4-chinolylketon vom Fp. 113—116°C.

Beispiel I

In das Gemisch aus 170 Volumenteilen tert.-Butanol, 11,4 Teilen 2-Pyridyl-2-trifluormethyl-6,8-dichlor-4-chinolin-cyanomethan (Beispiel 9) und 3,5 Teilen Kalium-tert.-butylat wird unter intensiver Durchmischung bei 60°C Luft eingeleitet. Nach beendeter Reaktion setzt man 3,0 Teile Eisessig zu und verdünnt das Reaktionsgemisch mit 300 Volumenteilen Wasser. Das ausgefallene Produkt wird abgesaugt, mit Wasser gewaschen und im Vakuumtrockenschrank bei 50°C getrocknet. Man erhält 10,8 Teile = 80% d. Th. 2-Pyridyl-2-trifluormethyl-6,8-dichlor-4-chinolylketon vom Fp. 193—194°C.

Beispiel K

Man verfährt wie in Beispiel F beschrieben, setzt jedoch anstelle des 2-Pyridyl-2,8-bis-(trifluormethyl)-4-chinolin-cyanomethans 5,0 Teile 2-Pyridyl-2-trifluormethyl-6,8-dichlor-4-chinolin-cyanomethan (Beispiel 9) ein. Nach der gleichen Aufarbeitung erhält man 4,25 Teile = 87,5% d. Th. 2-Pyridyl-2-trifluormethyl-6,8-dichlor-4-chinolylketon.

**Patentansprüche**

1. 4-Chinolinmethan-Derivate der Formel I

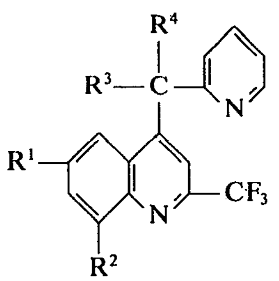

(I)

worin

$R^1$ ein Wasserstoffatom oder ein Chloratom
$R^2$ eine Trifluormethylgruppe oder ein Chloratom,
$R^3$ eine Cyano- oder Triphenylphosphoranmethino-Gruppe und
$R^4$ ein Wasserstoffatom oder einen Acetalrest mit bis zu 8 Kohlenstoffatomen

darstellen.

2. Verfahren zur Herstellung der 4-Chinolinmethan-Derivate der Formel I gemäß Anspruch 1, dadurch gekennzeichnet, daß man Verbindungen der Formel II

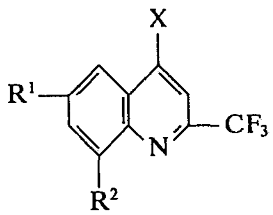

(II)

in der $R^1$ und $R^2$ die oben angegebene Bedeutung haben und in der X eine elektrofuge Gruppe bedeutet, mit einer Verbindung der Formel III

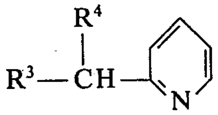

(III)

in der $R^3$ und $R^4$ die oben angegebenen Bedeutungen haben, in einem inerten Lösungsmittel in Gegenwart einer Base umsetzt.

3. Verfahren zur Herstellung von 2-Pyridyl-4-chinolylketonen der Formel IV

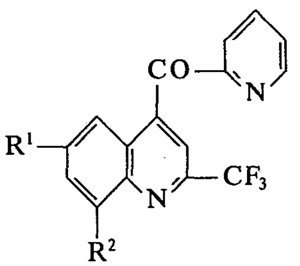

(IV)

worin $R^1$ und $R^2$ die angegebene Bedeutung besitzen, dadurch gekennzeichnet, daß man ein 2-Pyridyl--4-chinolin-cyanomethan der Formel V

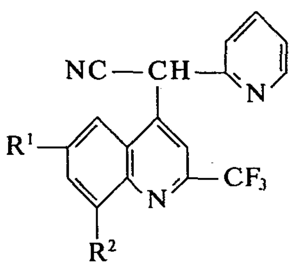

(V)

worin $R^1$ und $R^2$ die angegebene Bedeutung besitzen,

a) in polaren wasserfreien Medien in Gegenwart von Basen unter Begasen mit Sauerstoff oder sauerstoffhaltigen Gasen bei Raumtemperatur oder erhöhter Temperatur gegebenenfalls als Salz reagieren läßt oder
b) in saurem Medium mit einem Oxidationsmittel behandelt.

4. Verfahren zur Herstellung von 2-Pyridyl-4-chinolylketonen der Formel IV gemäß Anspruch 3, dadurch gekennzeichnet, daß man aus einem 2-Pyridyl-4-chinolin-cyanomethan der Formel VI

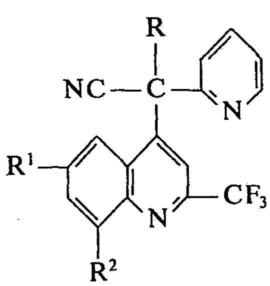

(VI)

worin $R^1$ und $R^2$ die angegebene Bedeutung besitzen und R einen Acetalrest mit bis 8 Kohlenstoffatomen bedeutet, den Acetalrest mit Säure abspaltet und das so erhaltene Cyanhydrin mit Alkali behandelt.

## Claims

1. A quinol-4-ylmethane derivative of the formula I

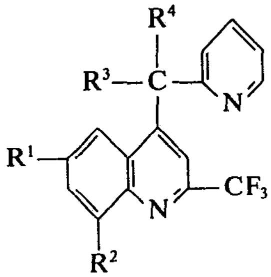

(I)

where $R^1$ is hydrogen or chlorine, $R^2$ is trifluoromethyl or chlorine, $R^3$ is a cyano or triphenylphosphoranemethino group, and $R^4$ is hydrogen, or an acetal radical of up to 8 carbon atoms.

2. A process for the preparation of a quinol-4-ylmethane derivative of the formula I as claimed in claim 1, wherein a compound of the formula II

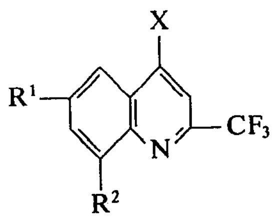

(II)

where $R^1$ and $R^2$ have the above meanings and X is an electrofugic group, is reacted with a compound of the formula III

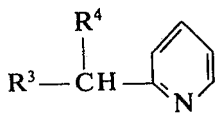

(III)

where $R^3$ and $R^4$ have the above meanings, in an inert solvent in the presence of a base.

3. A process for the preparation of a pyrid-2-yl-quinol-4-yl ketone of the formula IV

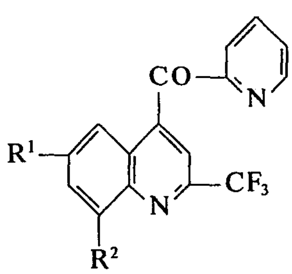

(IV)

where $R^1$ and $R^2$ have the above meanings, wherein a pyrid-2-yl-4-quinoline-cyanomethane of the formula V

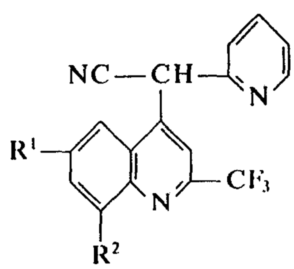

(V)

where $R^1$ and $R^2$ have the above meanings,

(a) is allowed to react, as such or in the form of a salt, in a polar anhydrous medium in the presence of a base, while gassing with oxygen or an oxygen-containing gas, at room temperature or elevated temperature, or
(b) is treated with an oxidizing agent in acid medium.

4. A process for the preparation of a pyrid-2-yl-quinol-4-yl ketone of the formula IV as claimed in claim 3, wherein the acetal radical is split off with acid from a pyrid-2-yl-4-quinoline-cyanomethane of the formula VI

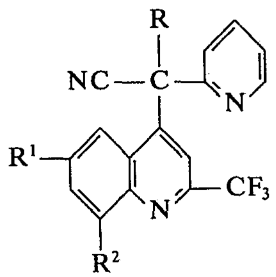

(VI)

where $R^1$ and $R^2$ have the above meanings, and R is an acetal radical of up to 8 carbon atoms, and the cyanohydrin thus obtained is treated with alkali.

**Revendications**

1. Dérivés du 4-quinoléine-méthane de la formule I

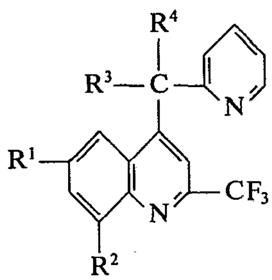

(I)

dans laquelle

$R^1$ désigne un atome d'hydrogène ou de chlore
$R^2$ désigne un groupe trifluorométhyle ou un atome de chlore
$R^3$ désigne un groupe cyano ou triphénylphosphorane-méthno
$R^4$ désigne un atome d'hydrogène ou un groupe acétal comportant jusqu'à huit atomes de carbone.

2. Procédé de préparation des dérivés de 4-quinoléine-méthane de la formule I selon la revendication 1, caractérisé en ce que l'on fait réagir des composés de la formule II

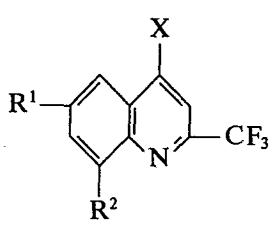

(II)

dans laquelle $R^1$ et $R^2$ possèdent la signification définie et X désigne un groupe électrofuge, dans un solvant inerte et en présence d'une base avec un composé de la formule III

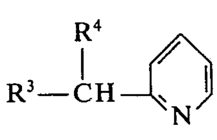

(III)

dans laquelle $R^3$ et $R^4$ possèdent les significations définies.

3. Procédé de préparation de 2-pyridyl-4-quinoléyl-cétones de la formule IV

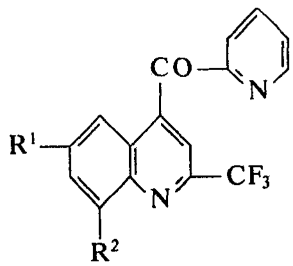

(IV)

dans laquelle $R^1$ et $R^2$ possèdent la signification définie, caractérisé en ce que l'on soumet un 2-pyridyl-4-quinoléine-cyano-méthane de la formule V

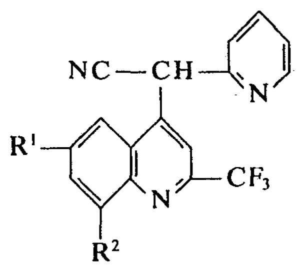

(V)

dans laquelle $R^1$ et $R^2$ possèdent la signification définie, soit

a) dans un milieu polaire anhydre, à la température ordinaire ou à une température accrue, à une réaction en présence de bases et avec barbotage d'oxygène ou de mélanges gazeux contenant de l'oxygène, le composé pouvant éventuellement être mis en oeuvre sous forme de sel, soit
b) dans un milieu acide à un traitement par un agent oxydant.

4. Procédé de préparation de 2-pyridyl-4-quinoléyl-cétones de la formule IV selon la revendication 3, caractérisé en ce que l'on provoque, à l'aide d'un acide, le clivage du groupe acétal d'un 2-pyridyl-4-quinoléine-cyano-méthane de la formule

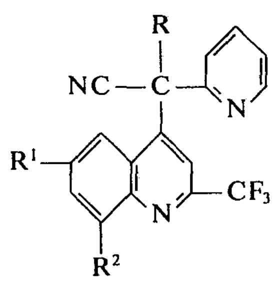

(VI)

dans laquelle $R^1$ et $R^2$ possèdent la signification définie et R désigne un groupe acétal comportant jusqu'à huit atomes de carbone, puis on traite la cyanhydrine obtenue avec un alcali.